Europäisches Patentamt

European Patent Office

Office européen des brevets

(11) Publication number: **0 172 209**
**B1**

## EUROPEAN PATENT SPECIFICATION

(45) Date of publication of patent specification: **10.05.89**

(51) Int. Cl.⁴: **G 01 N 1/20**

(21) Application number: **85901009.2**

(22) Date of filing: **05.02.85**

(86) International application number:
**PCT/FI85/00010**

(87) International publication number:
**WO 85/03572 15.08.85 Gazette 85/18**

(54) PROCEDURE AND MEANS FOR PRODUCER-SPECIFIC MILK SAMPLING.

(30) Priority: **06.02.84 FI 840481**

(43) Date of publication of application:
**26.02.86 Bulletin 86/09**

(45) Publication of the grant of the patent:
**10.05.89 Bulletin 89/19**

(84) Designated Contracting States:
**BE DE FR GB NL SE**

(56) References cited:
**EP-A-0 117 977**
**DE-A-3 222 234**
**DE-B-2 614 787**
**DK-A- 86 749**
**DK-A- 105 622**
**FI-B- 65 672**
**SE-B-81 030 660**
**US-A-3 163 047**
**US-A-4 022 066**

(73) Proprietor: **HACKMAN-MKT OY**
**Kuussillantie 18**
**SF-01230 Vantaa (FI)**

(72) Inventor: **SANTASALO, Hannu**
**Huuvaniementie 14 A 14**
**SF-00350 Helsinki (FI)**

(74) Representative: **Grams, Klaus Dieter, Dipl.-Ing.**
**et al**
**Patentanwaltsbüro Tiedtke-Bühling-Kinne-**
**Grupe-Pellmann-Grams-Struif Winter-Roth**
**Bavariaring 4**
**D-8000 München 2 (DE)**

## Description

The present invention concerns a procedure and an arrangement for producer-specific milk sampling according to the pre-characterizing portion of claim 1 respectively claim 5.

One of the problems to producer-specific sampling is how to obtain a sufficiently reliable and representative sample of the milk to be examined. It is important, in the quality assessment of the milk sold by the producer to the dairy, that the sample represents specifically the milk of the producer in question and in that state in which the milk is handed over from the producer to the dairy.

It is furthermore of importance that the sample is not affected by the quality of milk of any other producer, nor by the condition or deficient hygiene of the apparatus used by the dairy for sampling or milk collection.

The point at which the milk is handed over from the producer to the dairy can be imagined as being located at the end of a discharge connector of a farm milk tank in farm tank collection, and at the end of a suction pipe of a suction apparatus in can collection.

A procedure is in common use nowadays in which the sample is taken from carefully mixed milk from the farm tank with sterile implements into a sterile container. However, problems that occur are: poorly mixed milk, bacteria present in the discharge connector and which are not represented in the sample, and the task of keeping the sampling implement clean all through the long collection tour.

In farm tank collection, the milk in the discharge connector of the farm milk tank and in the immediate vicinity of a shut-off valve has significance in view of the milk's bacteria content, because the milk in the discharge connector is usually difficult to cool properly and the shut-off valve is often the part of the farm milk tank which is most difficult to clean.

A drawback of a sampling means accommodated in the measuring apparatus space of the tank or milk collection lorry is that it cannot be cleaned and sterilized other than at certain washing sites, usually in the dairy. Milk from other producers, and bacteria present in such milk, remain particularly in the suction tube used in milk collection. Hereby, the samle taken from the measuring apparatus space does not represent the same batch of milk which was just pumped through the measuring apparatus because the milk batches from other producers distort the representativeness of the sample taken from the milk batch of the next producer. The apparatus in the measuring apparatus space of the lorry also presents the drawback that bacertia may grow in the suction tube and contaminate the sample.

It has been found in practice that the milk composition varies in a stratified manner in the farm milk tank of the milk producer, even though the farm milk tank is provided with a mixer. Since the fat, protein and bacteria contents of the milk determine its price, samples must be taken for analysis of composition and for microbiological analysis. However, the stratification of the milk creates problems in this sampling.

DK—A—86 749 discloses an arrangement in which, for taking a sample, milk of the same milk batch is circulated from a container via a pump and a sampling means back into the container. In this arrangement a discharge element is followed by a suction tube, on which a sampling means is provided.

WO 82/04127 shows a sampling means completely integrated in a tank lorry. Between the sampling means and a discharge element of a milk container a suction tube belonging to the suction means is arranged.

DE—A1—2 614 787 shows a sampling arrangement serving to pick up samples from a liquid circuit.

DE—A1—3 222 234 shows a sampling means which is arranged in a parallel branch to a milk collection pipe.

DK—A—105 622 shows a procedure and an arrangement for milk sampling, wherein the milk is discharged by a milk pump from a milk container through a discharge element in the form of a suction pipe, then through a suction tube and a subsequent fixed tube into a collection tank. Upstream of the pump at the fixed tube a sampling means is provided which at this point takes a sample of the milk discharged from the milk container.

Due to the fact that in this procedure and in this arrangement, resp., the sampling means is disposed directly forward of the milk pump on the fixed tube, it is possible that the milk sample taken by the sampling means is not characteristic of the milk discharged from the milk container, for during passing through the suction tube, which is usually comparatively long, the milk is exposed to the most various influences by which its properties and its quality may be changed.

The subject matter of the invention is based on the object to further develop the last-mentioned procedure and arrangement, so that the milk samples taken correspond to the actual properties and the actual quality of the milk, which was discharged from a certain container during the sampling time, wherein influences occurring due to pumping process and changing the properties and the quality of the milk are largely to be eliminated.

This problem is solved, according to the invention, by the features contained in the characterizing portion of procedure claim 1 and arrangement claim 5, respectively. Due to the inventional arrangement of the sampling means it is possible to obtain a characteristic milk sample for each container and each container group, resp. The prior suction of milk from other containers has no impact on the respective newly collected milk sample.

A fixedly installed sampling means has been designed which in farm tank collection is placed in ths discharge connector of the farm tank after the shut-off valve, and between the suction pipe and milk drawing suction tube in milk can collection. The sampling means automatically separates from the milk a representative sample during the pumping. In modern milk collecting lorries it is possible to wash the suction pipe after each use.

The sampling means consists of a cylindrical body component fixedly mounted on the farm milk tank, a sampling nipple and a sample jar which can be hermetically attached to the sampling nipple or to the body component. The construction of the sampling means enables easy cleaning in connection with normal washing of the farm milk tank. The hygiene of the body component is the producer's responsibility. The end of the sampling means connected to the farm milk tank is constructed so as to fit to all farm milk tanks in use, and it is provided with suitable fixing elements. The end of the sampling means towards the suction tube is similar in construction to the normal discharge connector of the farm milk tank, and the suction tube can be connected thereto as to the normal discharge connector.

A sampling nipple serves as sampling means proper. One sampling nipple may be used several times, provided that it is cleaned well enough between uses. The person starting on his/her way to perform the sampling is given sample jars, which have been marked in advance, and a sufficient number of clean sampling nipples.

The person taking the samples mounts the sterile parts he/she has with him/her at the sampling point. The sampling nipple and jar are hermetically sealed to the body component of the sampling means for the duration of sampling. On concluded sampling, the sample jar is hermetically sealed and placed in a particular transport receptacle, and the sampling nipple is placed in the collecting container for potential later cleaning.

If it is desired to reuse the sampling nipples, they are washed, packed in plastic bags and sterilized in an autoclave for later use. The sample jars may be disposable, or they too are washed and sterilized.

By the procedure and means of the invention, for instance the following advantages are gained:

the sample is taken in the "right" spot at the "right" time,

the milk of other producers cannot affect the sample that has been taken,

all samples are proportional to the milk quantity, the use is simple,

the person taking the sample has no influence on the representativeness of the sample,

the procedure implies no great investment, operations are not tied to "collection route thinking",

the operation is simple and therefore reliable,

the milk supplier and the receiver can both establish that the sampling is done from the "proper" milk,

sampling takes place uniformly from the whole milk batch.

The invention is described in detail, reference being made to some advantageous embodiments presented in the figures of the drawing attached.

Fig. 1 presents the procedure of the invention in schematic elevational view as applied in farm tank collection.

Fig. 2 presents the procedure of the invention in schematic elevational view as applied in can collection.

Fig. 3 presents a detail of the arrangement of the invention on enlarged scale in partly sectioned elevational view.

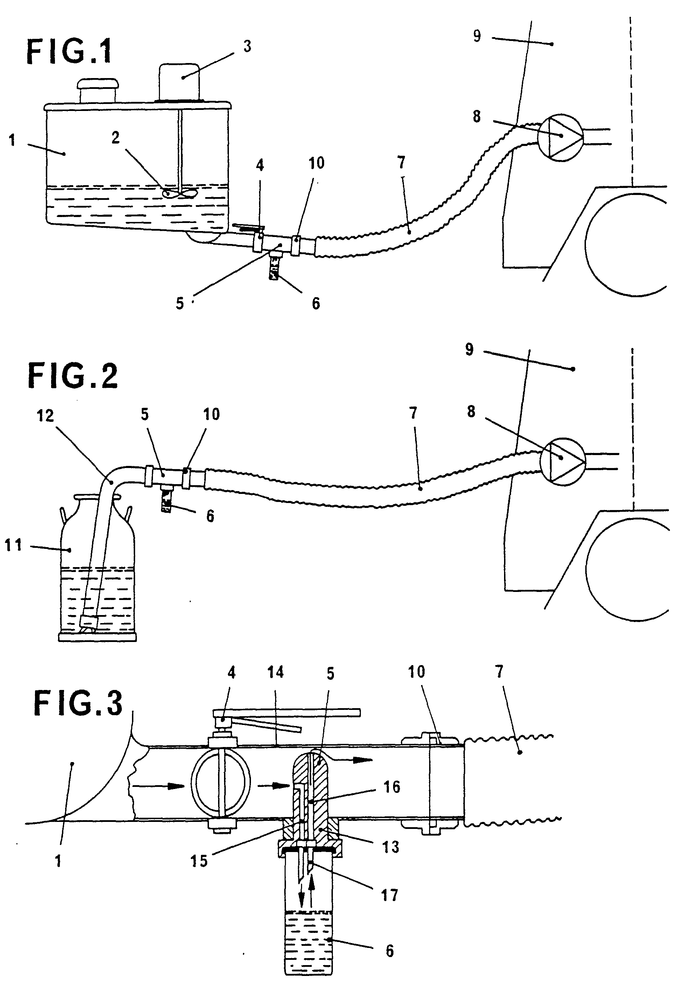

In the embodiment of Fig. 1, a milk container respectively a farm milk tank is indicated by reference numeral 1. A motor 3 has been disposed to rotate a mixing means 2 inside the farm milk tank 1. From the farm milk tank 1, a suction tube 7 leads to a tank lorry 9. A milk pump is indicated by reference numeral 8.

A sampling means 5 is placed between a shut-off valve 4 and the suction tube 7. The sampling means 5 is provided with a sampling jar 6.

In the embodiment of Fig. 2, the portable milk container, or can, is indicated by reference numeral 11. A milk suction pipe is indicated by reference numeral 12 and the suction tube to the tank lorry 9 by reference numeral 7, as in the embodiment of Fig. 1. The sampling means 5 has been placed between the suction pipe 12 and the milk drawing section tube 7. By refreence numeral 10 is indicated a coupling connector for the suction tube 7.

In Fig. 3 is presented a more detailed construction of the sampling means 5. The sampling means 5 comprises a body component 14 and a sampling nipple 13, to which the sampling jet 6 is attached. The sample taken from the milk is disposed to run into the sampling jar 6 along a first tubular connector 15 on the sampling nipple 13. The air in the sampling jar 6 and that entering with the milk flows along a second tubular connector 16 from the sampling jar 6 through the sampling nipple 13 to the cylindrical body component 14, and further along the suction tube 7 onwards. By reference numeral 17 are indicated sampling needles, if any.

The sampling means 5 is placed on a discharge connector of the farm milk tank 1 after the shut-off valve 4, respectively in can collection between the suction pipe 12 and the suction tube 7. The sampling means 5 is arranged to separate automatically a representative sample from the milk during the pumping. The sampling means 5 consists of the body component 14 fixedly mounted on the farm milk tank 1, the sampling nipple 13 and the sample jar 6 hermetically connectable to the sampling nipple 13 and/or the body component 14. When no sample is taken, the hole in the body component 14 for the sampling nipple 13 is kept closed with a plug or equivalent.

The body component 14 of the sampling means 5 is tank-specific and permanently mounted in place in farm milk tank collection. Its design allows easy cleaning in connection with the normal washing of the farm milk tank 1. The end of the sampling means 5 towards the farm milk tank 1 is so designed that it fits all farm milk tanks in current use, and it is provided with suitable fixing elements. The end of the sampling means 5 towards the suction tube 7 is equivalent in structure to the normal discharge connector of the farm milk tank 1, and a collector tube can be connected to it in like manner as to a normal discharge connector.

In the procedure respectively the arrangement of the invention, the sampling nipple 13 serves as actual sampling member. Repeated use of one sampling nipple 13, provided that it is sufficiently cleaned in between, is possible.

A person doing the sampling is given pre-marked sample jars 6 and a sufficient number of sampling nipples 13. The person mounts the hygienic parts he/she has with him/her at the sampling point. The sampling nipple 13 and the sampling jar 6 are hermetically sealed to the body component 14 for the duration of sampling. After the sampling process, the sampling jar 6 is hermetically closed and placed in a particular transport receptacle, and the sampling nipple 13 is placed in the collecting container for potential later cleaning in the dairy.

In the foregoing, only some embodiments of the invention have been described and it is obvious to a person skilled in the art that numerous modifications can be made within the scope of the inventive idea presented in the claims following below.

## Claims

1. A procedure for producer-specific milk sampling by a sampling means (5) from a milk container (1; 11), which is left by the milk through a discharge element (4; 12) during drawing the milk from said milk container (1; 11) through said discharge element (4; 12) and along a suction tube (7) to a tank lorry (9) by a milk pump (8), characterized in that milk sampling is performed between said discharge element (4, 12) and said suction tube (7).

2. A procedure according to claim 1, wherein said discharge element (4) is a shut-off valve (4).

3. A procedure according to claim 1, wherein said discharge element (12) is a suction pipe (12).

4. A procedure according to one of the claims 1 to 3, wherein an exchangeable, disposable or cleanable and repeatedly usable sampling nipple (13) is used for milk sampling.

5. An arrangement for producer-specific milk sampling by a sampling means (5) from a milk container (1; 11) having a discharge element (4; 12) through which the milk is drawn by a milk pump (8) along a suction tube (7) to a tank lorry (9), characterized in that said sampling means (5) is arranged between said discharge element (4; 12) and said suction tube (7).

6. An arrangement according to claim 5, wherein said discharge element (4) is a shut-off valve (4).

7. An arrangement according to claim 5, wherein said discharge element (12) is a suction pipe (12).

8. An arrangement according to one of the claims 5 to 7, wherein said sampling means (5) comprises a body component (14), a sampling nipple (13) and a sampling jar (6) attachable to said sampling nipple (13) and/or said body component (14).

9. An arrangement according to claim 8, wherein said body component (14) of said sampling means (5) is fixedly mounted on said shut-off valve (4) of said milk container (1).

10. An arrangement according to claim 8, wherein said body component (14) of said sampling means (5) is fixedly mounted on said suction pipe (12).

11. An arrangement according to one of the claims 8 to 10, wherein on said sampling nipple (13) is disposed a first tubular connector (15), along which the milk flows to said sampling jar (6), and a second tubular connector (16), along which air in the sampling jar (6) and air entering with the milk flows out of said sampling jar (6) to said body component (14) and, further, along said suction tube (7) onward.

12. An arrangement according to one of the claims 8 to 11, wherein said sampling nipple (13) is exchangeable, disposable or cleanable and reusable.

## Patentansprüche

1. Verfahren für erzeugerspezifische Milchprobeentnahme mittels eines Probenahmeteils (5) aus einem Milchbehälter (1; 11), den die Milch durch ein Auslaßteil (4; 12) verläßt, während sie durch das Auslaßteil (4; 12) und längs einer Saugleitung (7) mittels einer Milchpume (8) aus dem Milchbehälter (1; 11) zu einem Tankwagen (9) gesaugt wird, dadurch gekennzeichnet, daß die Milchprobenentnahme zwischen dem Auslaßteil (4; 12) und der Saugleitung (7) durchgeführt wird.

2. Verfahren nach Anspruch 1, bei dem das Auslaßteil (4) ein Absperrventil (4) ist.

3. Verfahren nach Anspruch 1, bei dem das Auslaßteil (12) ein Saugrohr (12) ist.

4. Verfahren nach einem der Ansprüche 1 bis 3, bei dem ein austauschbarer, einmal verwendbarer oder reinig- und wiederholt verwendbarer Probenahmenippel (13) für die Milchprobeentnahme verwendet wird.

5. Anordnung für erzeugerspezifische Milchprobeeninahme mittels eines Probenahmeteils (5) aus einem Milchbehalter (1; 11), das ein Auslaßteil (4; 12) aufweist, durch das die Milch mittels einer Milchpumpe (8) längs einer Saugleitung (7) zu einem Tankwagen (9) gesaugt wird, dadurch gekennzeichnet, daß das Probenahmeteil (5) zwischen dem Auslaßteil (4; 12) und der Saugleitung (7) angeordnet ist.

6. Anordnung nach Anspruch 5, bei der das Auslaßteil (4) ein Absperrventil (4) ist.

7. Anordnung nach Anspruch 5, bei der das Auslaßteil (12) ein Saugrohr (12) ist.

8. Anordnung nach einem der Ansprüche 5 bis 8, bei der das Probenabmeteil (5) ein Gehäuseteil (14), einen Probenahmenippel (13) und ein Probenahmegefäß (6) aufweist, das am Probenahmenippel (13) und/oder am Gehäuseteil (14) befestigbar ist.

9. Anordnung nach Anspruch 8, bei der das Gehäuseteil (14) des Probenahmeteils (5) fest am Absperrventil (4) des Milchbehälters (1) montiert ist.

10. Anordnung nach Anspruch 8, bei der das Gehäuseteil (14) des Probenahmeteils (5) fest am Saugrohr (12) montiert ist.

11. Anordnung nach einem der Ansprüche 8 bis 10, bei der am Probenahmenippel (13) eine erste Röhrenverbindung (15), längs der die Milch in das Probenahmegefäß (6) fließt, und eine zweite Röhrenverbindung (16) vorgesehen sind, längs der Luft im Probenahmegefäß (6) und mit der Milch eintretende Luft aus dem Probenahmegefäß (6) zum Gehäuse (14) und, danach, längs der Saugleitung (7) weiterfließt.

12. Anordnung nach einem der Ansprüche 8 bis 11, bei der der Probenahmenippel (13) austauschbar, einfach verwendbar oder reinig- und wiederverwendbar ist.

## Revendications

1. Procédé d'échantillonnage du lait spécifique à un producteur par un moyen d'échantillonnage (5) à partir d'un conteneur à lait (1; 11), à partir duquel le lait est évacué par un élément de décharge (4, 12) pendant le soutirage du lait à partir du conteneur à lait (1; 11) à travers ledit élément de décharge (4; 12) et le long d'une conduite d'aspiration jusqu'à un camion citerne (9) par une pompe à lait (8), caractérisé en ce que l'échantillonnage de lait est effectué entre l'élément de décharge (4; 12) et ledit tube d'aspiration (7).

2. Procédé selon la revendication 1, caractérisé en ce que l'élément de décharge (4) est une vanne d'arrêt (4).

3. Procédé selon la revendication 1, caractérisé en ce que l'élément de décharge (12) est une conduite d'aspiration (12).

4. Procédé selon l'une des revendications 1 à 3, caractérisé en ce qu'un mamelon d'échantillonnage (13) interchangeable, jetable ou nettoyable et utilisable à plusieurs reprises est utilisé pour l'échantillonnage du lait.

5. Agencement pour l'échantillonnage du lait spécifique à un producteur par un moyen d'échantillonnage (5) à partir d'un conteneur à lait (1; 11) ayant un élément de décharge (4; 12) à travers lequel le lait est soutiré par une pompe à lait (8) le long d'une conduite d'aspiration (7) vers un camion citerne (9), caractérisé en ce que ledit moyen d'échantillonnage (5) est disposé entre ledit élément de décharge (4; 12) et ledit tube d'aspiration (7).

6. Agencement selon la revendication 5, caractérisé en ce que l'élément de décharge (5) est une vanne d'arrêt (4).

7. Agencement selon la revendication 5, caractérisé en ce que l'élément de décharge (12) est une conduite d'aspiration (12).

8. Agencement selon l'une des revendications 5 à 7, caractérisé en ce que le moyen d'échantillonnage (5) comprend un composant de corps (14), un mamelon d'échantillonnage (13) et un récipient d'échantillonnage (6) pouvant être fixé sur ledit mamelon d'échantillonnage (13) m et/ou sur ledit composant de corps (14).

9. Agencement selon la revendication 8, caractérisé en ce que ledit composant de corps (14) dudit moyen d'échantillonnage (5) est monté à demeure sur la vanne d'arrêt (4) du conteneur à lait (1).

10. Agencement selon la revendication 8, caractérisé en ce que le composant de corps (14) du moyen d'échantillonnage (5) est monté à demeure sur la conduite d'aspiration (12).

11. Agencement selon l'une des revendications 8 à 10, caractérisé en ce que sur ledit mamelon d'échantillonnage (13) est agencé un premier raccord tululaire (15) à travers lequel s'écoule le lait vers le récipient d'échantillonnage (6) et un second raccord tubulaire (16) à travers lequel l'air dans le récipient d'échantillonnage (6) et l'air pénétrant avec le lait s'écoulent du récipient d'échantillonnage (6) vers le composant de corps (14) et ensuite par la conduite d'aspiration (7) et au-delà.

12. Agencement selon l'une des revendications 8 à 11, caractérisé en ce que ledit mamelon d'échantillonnage (13) est interchangeable, jetable ou nettoyable et réutilisable.

FIG.1

FIG.2

FIG.3

1